# EUROPEAN PATENT APPLICATION

(11) **EP 2 119 436 A1**
(43) Date of publication of application: **18.11.2009**
(21) Application number: 06849602.5
(22) Date of filing: 20.10.2006
(51) Int. Cl.: A61K 31/122, A61K 38/03, A61K 31/66, A61P 27/12, A61P 27/06, A61P 27/02, A61P 39/06

(54) **PHARMACEUTICAL COMPOSITIONS FOR PREVENTING AND TREATING EYE PATHOLOGIES**

(71) Applicant: Limited Liability Company "Mitotechnology", Moscow 119180 (RU)
(72) Inventor: SKULACHEV, Vladimir Petrovich, Moscow, 119234 (RU); SKULACHEV, Maxim Vladimirovich, Moscow, 119234 (RU)
(74) Representative: HOFFMANN EITLE
(86) International application number: PCT/RU2006/000546
(87) International publication number: WO 2008/048134

(57) **Abstract**

The invention relates to pharmacology, medicine and opthalmology, in particular to chemical compounds having a structure (I) and to solvates, isomers or pro-pharmaceutical forms thereof used in pharmaceutical compositions which contain pharmaceutically acceptable carrier and can be useful for preventing and treating different eye pathologies, in particular cataract and macular dystrophy.

## Description

### Field of the Invention

The present invention relates to pharmacology, medicine, ophthalmology and deals with a class of chemical compounds having a structure (1), in particular, which can be applied in the makeup of pharmaceutical compositions for preventing and treating various eye pathologies, specifically cataract and macular dystrophy.

### State of the Art

By now there exists a wide variety of methods of treatment of eye pathologies in medical practice - - surgical and medicamental, the latter being as a rule represented by natural and synthetic compounds having antioxidant properties (antioxidants).

In the pathogenesis of many eye pathologies (cataract and macular dystrophy included), of substantive importance is oxidation stress - - unbalance between the production of free radicals and their elimination by antioxidants.

Until now in Russia available were only bioactive additives (BAA) which contain carotinoids of natural origin having antioxidant properties - lutein and zeaxanthin. Besides, a plant carotinoid- - beta-carotene enters into the composition of preparations (1.5 mg) to play an important role in building an eye pigment - rodopsin providing eye adaptation to reduced illumination; antioxidants - vitamins E and C, microelements - zinc and copper which are likewise important for providing the health of eyes.

Among the diseases leading to a steady weakening of eye functions and blindness, age-related macular degenerations (AMD) occupy one of the leading positions in the world (J.Evans, C. Rooney, F. Ashood, 1996). AMD - one of the most complex eye diseases for treatment (L.N. Marchenko, 2001). According to the data of the Central Institute for examination of workability and invalidism for 1997, among the vascular diseases of the retina necessitating eye-sight invalidation, non-exudative forms of age-related macular degeneration comprise 39.4%, exudative - - 9.1%, disturbance of circulation in the main vessels of the retina - 51.5%. Some 25 years ago the age-related macular degeneration in West Europe was about 10% of the registered blindness. Now this figure has grown up to 50% (V.S. Akopian, 2004). A trend to the predominance of elderly persons among the population results (WHO data for 1986) in the annual growth of a sick rate of the given pathology. The history of a problem of macular degeneration originates from 1855 when F.S. Donders described macular druses. The term "bluing macular degeneration" was first used by O.Haab in 1885. Further, C.Behr (1920) and H.F. Falls (1949) referred said pathology to hereditary familial diseases. Considering a variety of clinical and opthalmoscopic data, the age-related macular degeneration was described in foreign and national literature under diverse terms. By now ophthalmologists are unanimous in that all these kinds of pathology are a manifestation of one disease that is now defined in world literature more often than not as "age-related macular degeneration" (AMD).

AMD is the pathology of the central photoactive zone of the retina. The disease is a chronic dystrophic process with mostly affected choriocapillary layer, Bruck's membrane and a pigment retina epithelium (PRE), with photoreceptors involved (V.S. Lysenko and co-authors, 2001). A degree of severity of the process and loss of central vision depends on AMD form and proximity of the dystrophic process to the central fossa of the retina. AMD is more often a dual process. It has been found that the second eye is damaged not later than 5 years after the first (H.C. Zweng, 1977).

Given the progression of macular degeneration, there appears enhanced light sensitivity, vision deteriorates, vision sharpness is reduced, there gradually occurs prolapse of fields of vision and ultimately a turbid macula appears in the center of a field of vision (relative or absolute scotoma). The reasons behind the development of macular degeneration are varied. However, the role of genetic factors and impairing light effects leaves no room for doubt. Recently the problem of a role of the negative effect of free oxygen radicals is becoming more and more of a talking point in world scientific circles. A photochemical reaction originating when acted upon by light and oxygen brings about the formation of highly active free radicals which are capable of damaging the photoactive cells of the retina. The older the subject, the more dangerous is an effect of the free radicals - - with age-related senescence, activity of one's own protective antioxidant system of an organism is reduced, a factor that aggravates dystrophic processes.

Continuous operations with a computer likewise worsen vision. A computer monitor is a source of increased danger for the eyes, as radiating UV light whose effect is increased with the use of fluorescent lamps. In combination with the intense work of the eyes this is likely to cause quick fatigue, headaches, a sharp pain in the eyes, epiphora and to lower workability. Statistical data go to show that from 50 to 90% of subjects working with the computer make complaints to physicians which are united by the term - computer vision syndrome (CVS). For the antioxidant protection of eye-sight organs to be strengthened, the subjects who do a great deal of work in the computer need the additional doses of antioxidants.

Antioxidant-vitamins, such as vitamins C and E, bioflavonoids, beta-carotene protect the eyes from damage as well and promote restorative processes thereby to maintain collagen synthesis. The co-use of N-acetyl-cysteine, a-lipoic acid and vitamins C and E stimulates the synthesis of one of the basic antioxidant ferments of eye tissue-glutathione.

### Essence of the Invention

One of the aspects of the present invention is a pharmaceutical composition for preventing and treating various eye pathologies, including a compound comprising an addressing portion, a linker group and an antioxidant. The general chemical structure of such compounds has the following general formula (1): 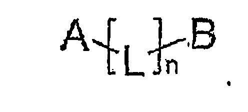 wherein A - effector group - antioxidant. 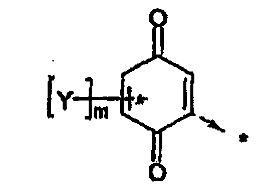 and/or its restored form
wherein m - integer from 1 to 3; Y - different or identical substituents representing lower alkyl or lower alkoxy; or two vicinal Y are so interlinked as to form the structure: 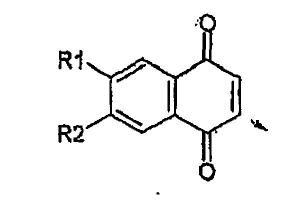 and/or its restored form
wherein R1 and R2 are different or identical substituents being each independently lower alkyl or lower alkoxy; L - linker unit representing:
a) single or branched hydrocarbon chain, optionally substituted with one or more substituents and, in case of necessity, comprising one or more
   double or triple bonds;
b) natural isoprenoid chain; n - integer from I to 20;
   B - represents
a) Skoulachev - ion Sk: Sk⁺ Z"
   wherein Sk - lipophilic cation;
   Z - pharmacologically acceptable anion;
b) charged hydrophobic peptide of 1-20 aminoacids
   except for the compounds in which A represents ubiquinone (2-methyl-4,5-dimethoxy-3,6-dioxo-1,4-cyclohexadienyl) or tocopherol or the mimetic of a superoxidedismutase or ebselen, besides that L - divalent decyl or divalent pentyl or divalent propyl and B - triphenylphosphomim; and also its solvates, isomers or prodrug forms.

Another aspect of the present invention is a pharmaceutical composition for preventing and treating various eye pathologies, including a compound of a structure (II), wherein A is a residue of plastoquinone of the formula: 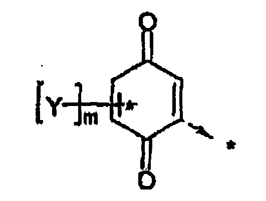 Y - methyl, m=2;
L - linker unit representing:
a) single or branched hydrocarbon chain, optionally substituted with one or more substituents and, if necessary, comprising one or more double or triple bonds;
b) natural isoprenoid chain;
   n - integer from 1 to 20;
   B - represents
a) Skoulachev - ion Sk: Sk⁺ Z"
   wherein Sk - lipophilic cation;
   Z - pharmacologically acceptable anion;
b) charged hydrophobic peptide of 1-20 aminoacids
   except for the compounds in which A represents ubiquinone (2-methyl-4,5-dimethoxy-3,6-dioxo-1,4-cyclohexadienyl) or tocopherol or the mimetic of a superoxidedismutase or ebselen, besides that L - divalent decyl or divalent pentyl or divalent propyl and B - triphenylphosphomim; as well as its solvates, isomers or prodrug forms.

A further aspect of the present invention is a pharmaceutical composition for preventing and treating various eye pathologies, including a compound of the structure (1) - SkQ1

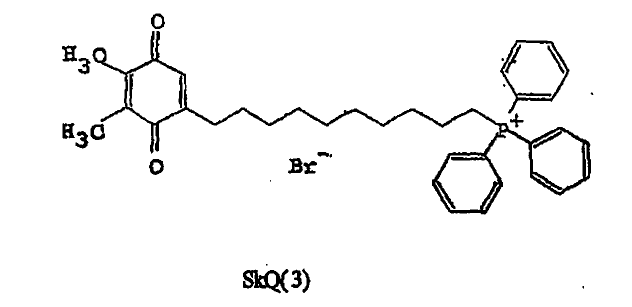

A still further aspect of the present invention is a pharmaceutical composition for preventing and treating various eye pathologies, including a therapeutically or prophylactically justifiable amount of a compound of the structure (1) and at least one pharmaceutically acceptable diluent or a carrier. The pharmaceutically acceptable diluent or carrier may be an excipient, a thinner (diluent) or a mixture thereof. By the "therapeutically justifiable" amount of a substance is implied an amount of compound (1) which provokes the desirable biological or medical response of a patient who undergoes treatment with a physician or a veterinarian. By the "prophylactically justifiable" amount of a substance is implied an amount of compound (1) which prevents or suppresses a disease or ameliorates a course of disease in the patient suffering a medical condition which the physician or the veterinarian tries to prevent, suppress or to ameliorate.

In one of the aspects of the present invention a patient is man.

"Eye pathologies" include but are not restricted to: various forms of macular degeneration (MD) and other sympthoms related thereto, namely: atrophic (dry) MD, exudative (wet) MD, age-related maculopathy (ARM), choroidal neovascularization, detachment of the pigment epithelium of the retina (PED), retina pigment epithelium atrophy (RPE). The notion "macular degeneration (MD)" also includes all eye diseases not relating to age-related changes in a human organism, namely: Best's disease or vitelliform, Stargardt's disease, juvenile macular dystrophy, Behr's disease, Sorbsby disease, alveolar dystrophy or Doin's disease. "MD-related symptoms" include but are not restricted to: druses surrounded by whitish-yellowish maculae, a submacular discal tissue rumen, choroidal neovascularization, PED, RPE, an abnormal growth of choroid blood vessels, a smeared or upset field of vision, a central deadblock, pigment anomalies, a fine granulation mixed layer disposed on the inner side of Bruck's membrane, a thickening and said membrane's reduced permeability.

Greatly helpful are compositions of the invention for treating and/or preventing such diseases as: cataract, particularly senile cataract, diabetic cataract; retina pathology (retinopathy). the detached retina, the pathology of retina vessels, eye choroid, optic nerve, specifically optic nerve artrophy, central and peripheral chorioretinal dystrophies, particularly uveits; intraocular hemorrhage; traumatic hemorrhage; conjunctivitis; eye sores; keratitis, particularly dry keratoconjunctivitis; glaucoma.

Causes for macular degeneration include but are not restricted to: genetic or physical traumas, diseases such as diabetes or infections, particularly bacterial.

Compounds of Formula I can be used for the effective prophylaxis or therapy of all forms of macular degeneration (MD) and other syndromes or symptoms associated therewith, regardless of causes thereof.

Use of pharmaceutical compositions relating to the present invention can be both somatic and local. Methods of administration include enteral such as oral, sublingual and rectal methods; local such as transdermal, intradermal and oculodermal, and parenteral methods. Acceptable parental methods of administration include injections, for example, intravenous, intramuscular, subcutaneous, intraperitoneal, endarterial injections, to mention only few, and noninjection methods, such as intravaginal or nasal. Preferably compounds and pharmaceutical compositions relating to the present invention are administered perocularly or perorally. In particular, administration can be in the form of eye drops or tablets, pellets, capsules or some other pressed or molded form.

On administration of a compound of Formula (I) in the form of a pharmaceutical composition, the compound of interest should be mixed according to a compounding formulation, with a suitable amount of a pharmaceutically acceptable diluent or carrier in such a way as to have an appropriate form to be administered to a patient. The term "diluent" is related to a thinning agent, an auxiliary drug, an excipient or a carrier with which the compound of interest is mixed to be administered to the patient. Said pharmaceutical carriers may be liquids, such as water and oils, including petroleum, animal, plant and synthetic oils, such as peanut oil, soybean oil, mineral oil, or the like. Pharmaceutical diluents may be physiological solutions, acacia gum, gelatin, starch, talc, keratin, colloidal silver, urea, etc.

Likewise, a composition may include auxiliaries, stabilizers, thickeners, lubricants and coloring agents.

Compounds and compositions relating to the present invention can be administred in the form of capsules, tablets, pills, pads, granules, syrups, elexirs,solutions, ophthalmological solutions, suspensions, emulsions, suppositories or substained release formulations or in some other form suitable to a patient. One of the aspects of the present invention is application of compounds of Formula (1) and compositions in the form of solutions for peroral and perocular administration.

The therapeutically justifiable amount of a compound of Formula (I) required for treating a particular disease or a symptom depends on the nature of the disease or on the symptom and a method of administration and should be ascertained by an attending physician. In principle, the acceptable doses of peroral administration are I to 500 mcg/kg, preferably 25 mcg/kg, and more preferably 125 mcg/kg of a patient's body weight.

### Example of acceptable pharmaceutical composition in the form of solution for peroral use

10 mM Na-phosphate buffer, pH 6.0
SkQ1 in a concentration of 125 mcg/ml
Aqueous solution

### Example of acceptable pharmaceutical composition in the form of ophthalmological solution (eye drops)

10 mM Na-phosphate buffer, pH 6.5
250 mM aqueous SkQ1 solution
0.9% aqueous NaCl solution

### Brief description of figures

Fig. 1 - influence exerted by preparations on OXYS rats suffering from cataract.
Fig. 2 - influence exerted by preparations on OXYS rats suffering from macular dystrophy.
Fig. 3 - influence exerted by preparations on severity of a cataract course of OXYS rats.
Fig. 4 - influence exerted by preparations on severity of a macular degeneration course of OXYS rats.

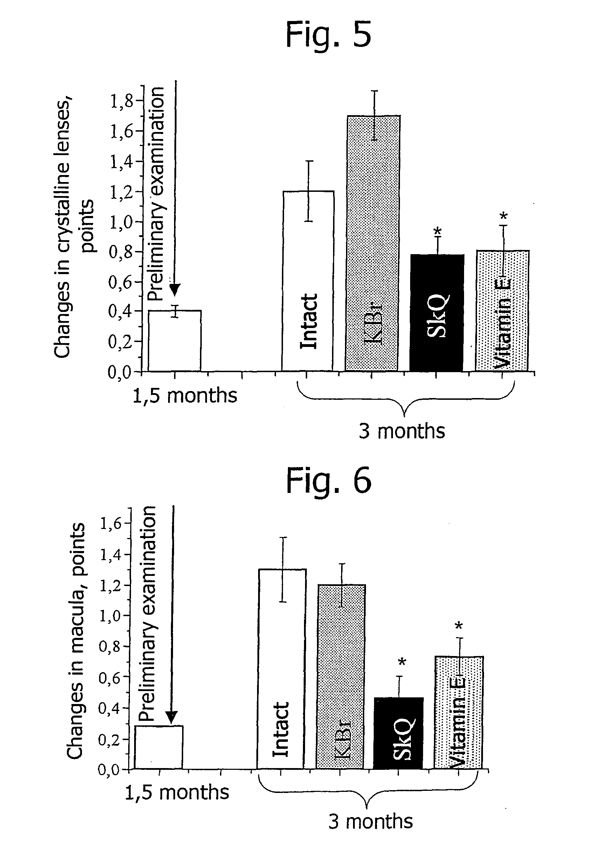

Fig. 5 - evaluation of the state of OXYS rats crystalline lenses before the ingestion of preparations and after a 45-day course of KBr, SkQ and vitamin E.
Fig. 6 -evaluation of degenerative changes in an OXYS rats retina macular area before an intake of preparations and after 45-day course of KBr, SkQ or vitamin E.
Fig. 7 - data on therapeutical SkQ1 effect on the state of OXYS rats crystalline lenses.
Fig. 8 - data on a therapeutical SkQ1 effect on the state of OXYS rats crystalline lenses.

### Execution of the Invention

Given below is a number of experimental examples to illustrate a possibility of an invention being executed, specifically the action of substances conforming to a structure (1) in accordance with the invention. These examples should only confirm soundness of the teachings and shouldn't be taken as limiting a field of use or application thereof.

### Prophylactic effect of SkQ1 antioxidant of invention in age-related eye diseases

Work was carried out on male rats (OXYS and Vistar). Animals were kept in cages, 5 species each with natural illumination and received a standard granular feed and water with no limit. At the age of 1.5 months, the rats were examined by an ophthalmologist upon a preliminary widening of pupils with 1% tropicamide. From 1.5 to 3 months at the critical age for the development of well-pronounced changes in the organ of vision of OXYS rats, part of the animals received SkQ (50 nM per kg of body weight), KBr (50 nM per kg) or vitamin E - alpha-tocopherol acetate ("Uralbiofarm") 20 mg per kg. The latter is traditionally used as a comparison preparation. The animals received preparations before meals on a rusk of standard size; intact control groups received the rusk alone. On completion of a course, the animals were weighed and re-examined. To avoid a subjective evaluation of intake results, all the inscriptions on the cages were removed beforehand.

Ophthalmoscopic investigations were carried out by means of an ophthalmoscope "Betta" (Germany). In part of fluotan narcotized animals (1-1.5 min.) the eyeground was photographed, fluorescent angiography was performed by means of a Fundus-camera "Opton" or crystalline lenses were inspected by means of a slit lamp "Opton" SL30 using a system of automatic image registration (biomicroscopic examination).

The state of crystalline lenses was assessed in accordance with a classification (in points) accepted in clinical practice (L.A. Katsnel'son, T.I. Forofonova, A. Ya. Bounin, 1990): 0 - crystalline lens, transparent; 1- focal gentle cloudiness; 2 - multiple foci of cloudiness; 3- intense cloudiness of the cortex or kernel of a crystalline lens. Presence and a well-pronounced degree of focal changes in a macular area were assessed under a universally adopted classification: 0 - no changes; 1 - 1^{st} stage of disease in which druses appear in the posterior pole of an eye; 2 - 2^{nd} stage, development in a macula and a paramacular area, of a prominent focus of yellow color with clear-cut contours dimensioned up to 0.5 to 1 of a disk dia. (exudative retinal pigment epithelium detachment) and 3 - 3^{rd} stage with ample hemorrhages into the macular area.

The results were statistically processed by means of a factor dispersion assay (ANOVA/MANOVA, Statistica, 5) with post hoc comparison of group means (Newman-Keul test), with a genotype and a preparation considered as independent factors.

### The results obtained:

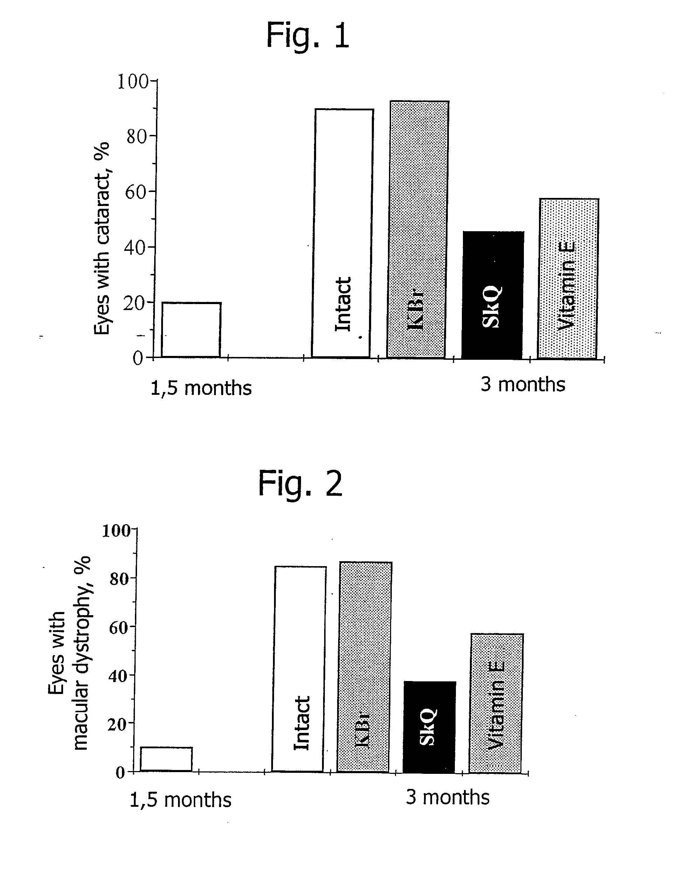

Ophthalmoscopic examination did not reveal changes in the crystalline lenses and macular area of the retina of Vistar rats (1.5 or 3 month age). At the same time in 1.5 months, 20% of the eyes of OXYS rats already had an initial stage of cataract (I point) (Fig. 1) and 10% - - macular degeneration, 1^{st} stage (Fig.2).

At the age of 3 months, in 90% of the eyes of intact OXYS rats there were revealed changes in crystalline lenses, particularly in 35% of the eyes, changes corresponded to the 2^{nd} stage of cataract, dominated by ring-shaped and kernel cataracts (Fig. 1).

Intact animals (85%) were affected by macular dystrophy, 16% of them - 2^{nd} stage (Fig. 2). In the group of OXYS rats which received KBr, 93% of the eyes had changes in the crystalline lenses, with 57% of the crystalline lenses (of the total of the eyes) having changes corresponding to the 2^{nd} stage of cataract. In 87% of the eyes of this group of rats there were revealed changes in the retinal macular area; in 13% of cases, retinal changes corresponded to the 2^{nd} stage of disease. Given the fact that 20% of the eyes were already affected by cataract, 73% of the rats got sick again; 77% affected by macular dystrophy respectively.

In 46% of the eyes of OXYS rats which received SkQ (Fig.1) there were revealed gentle changes in crystalline lenses corresponding to the 1^{st} stage. The changes of a macular retinal area were revealed in 38% of OXYS rats of this group, all of them also corresponding to the 1^{st} stage, as to degree (Fig. 2). Against a background of the preparation, some 26% of rats of this group were affected with cataract (2.8 times less than a KBr group): macular dystrophy - 28% (2.8 times less, too).

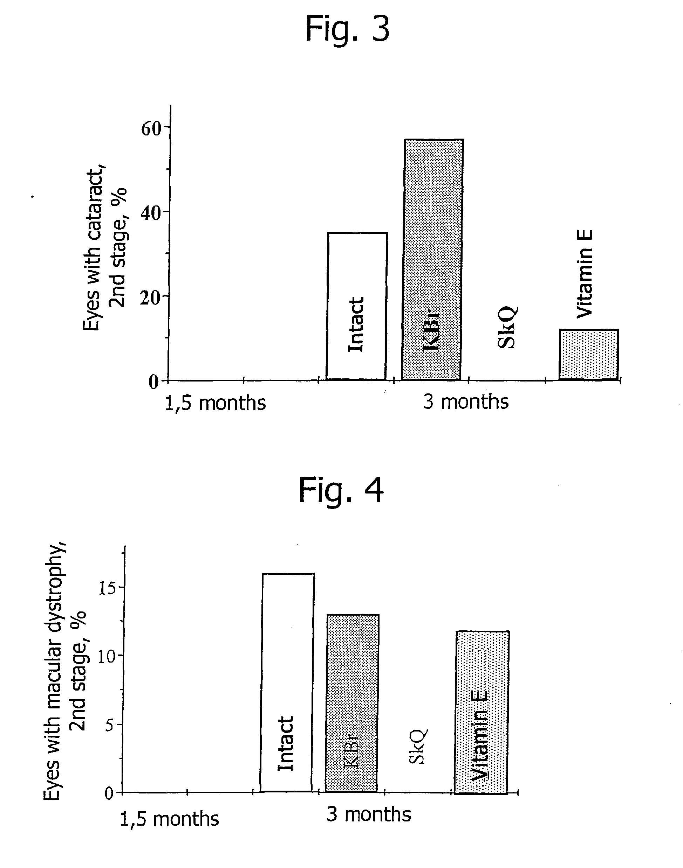

In rats which received vitamin E (3 months), changes in crystalline lenses were revealed in 58% of the eyes (an increase of 38%, 1.8 times less than in the case of intact rats), with 12% of the eyes having changes corresponding to the 2^{nd} stage of a disease. The changes of a macular retinal area were revealed in 54% of OXYS rats of this group (1.5 times less than in the case of intact rats) and more than that an eye disease corresponded to the 2^{nd} stage of macular dystrophy (8%). The intake of SkQ not only diminished the incidence of cataract and macular dystrophy but also significantly affected severity of their course (cf. Fig. 3 and Fig. 4).

It is noteworthy that one month after completion of an SkQ course, in SkQ rats, as distinct from the control animals, no changes occurred in the state of the retina and the crystalline lens.

It is hence only logical to deduce that investigations, as carried out, demonstrate a possibility of SkQ being used in the preventive treatment of the age-related diseases of an eye-sight organ, particularly cataract and macular dystrophy - - major age- dependent eye diseases in elderly people.

### Therapeutic effect of SkQ1 antioxidant of invention in case of age-related eye diseases

An experiment was carried out according to the same diagram as the above-described experiment exhibiting an SkQ1 prophylactic action, with the exception that used therein were Vistar and OXYS animals, aged 10.5 months.

Table 8 shows the examination results of animals before an intake of a preparation at the age of 10.5 months and after a course of treatment. Revealed in Vistar rats were changes of crystalline lenses and the retina exceeding the indices characteristic of the corresponding age, which fact is explained by the peculiarities of the upkeep of these animals in a vivarium. On inspection 45 days after, the state of crystalline lenses in the control rats (Vistar) remained intact; nor was it significantly affected by preparations. And among SkQ1 Vistar rats, the animals having changes in the retina which could be classified as corresponding to even an initial stage of disease were practically absent.

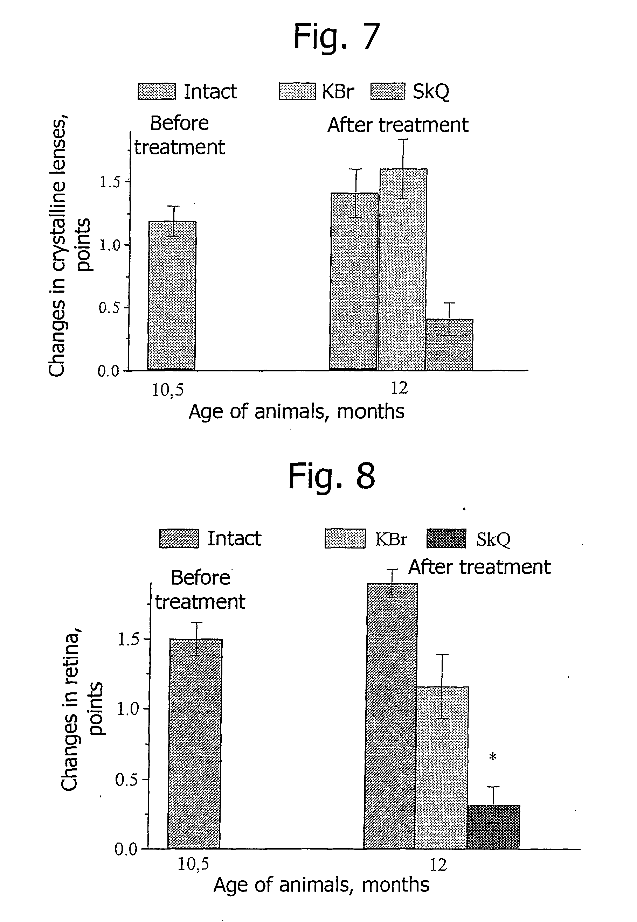

According to pair comparisons, in OXYS intact rats, the changes of crystalline lenses and the retina significantly intensified during 1.5 months (p < 0.04 and p < 0.01, respectively. In KBr animals significantly intensified were changes of the crystalline lenses while those of the retina remained intact. It should be noted that a comparison was made of a condition of the eyes before and after treatment; a comparison of mean values (Table I, Figs. 7, 8). In SkQ rats (OXYS), a degree of said well-pronounced changes was appreciably diminished indeed. Edemas markedly abated in the retina of animals of this group. The number and area of ischemia zones was reduced, and hemorrhages resolved.

**Table I**

| The state of animal's eyes before and after a SkQ course (50 nmol per kg of body weight). The table shows changes in crystalline lenses and a macular area of the retina (in points). | | | | |
|---|---|---|---|---|
| | Preliminary examination, 10,5 months | Inspection after course of treatment, 12 months | | |
| | | Intact | KBr | SkQ |
| | OXYS | | | |
| Macula | 1,46±1,18 | 1,90±0,10 | 1,13±0,17 | 0,32±0,13 |
| Crystalline lenses | 1,19±0,12 | 1,41±0,19 | 1,6±0,23 | 0,41±0,13 |
| | Vistar | | | |
| Macula | 0,48±0,19 | 0,5±0,25 | 0,25±0,10 | 0,09±0,06 |
| Crystalline lenses | 0,40±0,23 | 0,5±0,22 | 0,5±0,15 | 0,54±0,14 |

Data thus obtained indicate that a SkQ - based pharmaceutical composition produces therapeutic effects on animals suffering from cataract or retinopathy conforming to human macular dystrophy.

## Claims

1. A pharmaceutical composition for the treatment of eyes comprising a therapeutically effective amount of a compound of Formula I: 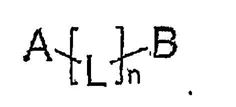 wherein A - effector group - antioxidant 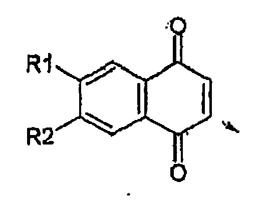 and/or its reduced form,
wherein m - integer of 1 to 3; Y - different or identical substituents representing lower alkyl or lower alkoxy; or two vicinal Y are so interlinked as to form a structure; 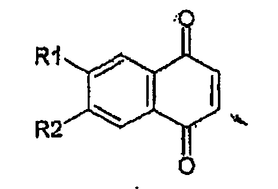 and/or its reduced form,
wherein R1 and R2 are different or identical substituents independently representing lower alkyl or lower alkoxy; L - linker unit representing:
a) single or branched hydrocarbon chain, optionally substituted with one or more substituents and, if necessary, having one or more double or triple bonds;
b) natural isoprenoid chain;
n - integer of I to 20;
B - represents
a) Skoulachev - ion Sk:
Sk⁺Z⁻
wherein Sk - lipophilic cation; Z - pharmacologically acceptable anion;
b) charged hydrophobic peptide of 1-20 amino acids,
with the exception of compounds in which A is ubiquinone (2-methyl-4,5-dimethoxy-3,6-dioxo-1,4-cyclohexadienyl) or tocopherol or the mimetic of a superoxidedismutase or ebselen, besides that L - divalent decyl or divalent pentyl or divalent propyl and B - triphenylphosphomim; and also its solvates, isomers or prodrug forms and a pharmacologically acceptable carrier.

2. The composition of claim 1 wherein A is a plastoquinone residue of the formula: 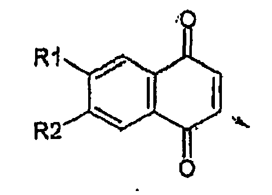 Y - methyl, m=2;
L - linker unit representing:
a) single or branched hydrocarbon chain, optionally substituted with one or more substituents and, if necessary, comprising one or more double or triple bond;
b) natural isoprenoid chain;
n - integer from 1 to 20;
B - representing
a) Skoulachev - ion Sk:
Sk⁺ Z"
wherein Sk - lipophilic cation;
Z - pharmacologically acceptable anion;
b) charged hydrophobic peptide of 1-20 amino acids,
with the exception of compounds in which A is ubiquinone (2-methyl-4,5-dimethoxy-3,6-dioxo-1,4-cyclohexadienyl) or tocopherol or the mimetic of a superoxidedismutase or ebselen, besides that L - divalent decyl or divalent pentyl or divalent propyl and B - triphenylphosphomim; as well as its solvates, isomers or prodrug forms and a pharmacologically acceptable carrier.

3. The composition of claim 2 wherein the compound of Formula I is a SkQ substance:

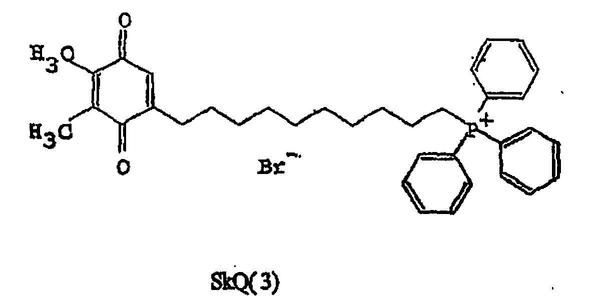

4. The composition of any one of claims 1-3 in the form of a solution for oral use.

5. The composition of any one of claims 1-3 in the form of an opthalmologic solution for perocular use.

6. The composition of any one of claims 1-3 in the form of a solution for parenteral use.

7. Use of a composition according to any one of claims 1-6 for preparing a medicinal for preventing and/or treating the eye pathologies of mammals.

8. Use according to claim 7, wherein a mammal is man.

9. Use according to claim 7, wherein the pathology is retinal macular dystrophy.

10. Use according to claim 7, wherein the pathology is cataract, particularly senile cataract, diabetic cataract.

11. Use according to claim 7, wherein the eye pathology of mammals is retinopathy, detached retina, retinal vessels pathology, an eye choroid, an optic nerve, particularly optic nerve atrophy, central and peripheral chorioretinal dystrophies, specifically uveits.

12. Use according to claim 7, wherein the pathology is intraocular hemorrhage, traumatic hemorrhage.

13. Use according to claim 7, wherein the pathology is conjunctivitis, eye sores, keratitis, dry keratoconjunctivitis.

14. Use according to claim 7, wherein the pathology is glaucoma.

15. A method of preventing and/or treating mammalian pathologies, comprising administering to a mammal in need thereof an effective amount of a pharmaceutical composition, as shown and described in one of claims 1-6.
